# EUROPEAN PATENT APPLICATION

(11) **EP 3 790 016 A1**
(43) Date of publication of application: **10.03.2021**
(21) Application number: 19796176.6
(22) Date of filing: 02.05.2019
(51) Int. Cl.: G16H 10/60, G16H 50/50

(54) **DISEASE NETWORK CONSTRUCTION METHOD CONSIDERING STRATIFICATION ACCORDING TO CONFOUNDING VARIABLE OF COHORT DATA AND OCCURRENCE TIME BETWEEN DISEASES, METHOD FOR VISUALIZING SAME, AND COMPUTER-READABLE RECORDING MEDIUM RECORDING SAME**

(30) Priority: 02.05.2018 KR 20180050851
(71) Applicant: CHA UNIVERSITY INDUSTRY-ACADEMIC COOPERATION FOUNDATION, Seongnam-si, Gyeonggi-do 13488 (KR)
(72) Inventor: HAN, Hyun Wook, Seongnam-si Gyeonggi-do 13559 (KR); YU, Jong Man, Seoul 06131 (KR); LEE, Dong Hyun, Seoul 04402 (KR); YUN, Ho, Yongin-si Gyeonggi-do 16904 (KR); HWANG, Tae Sun, Seongnam-si Gyeonggi-do 13597 (KR); LEE, Chaewon, Seoul 06689 (KR); KIM, Kanghyun, Yecheon-gun Gyeongsangbuk-do 36829 (KR); NAM, Sangmin, Seoul 02870 (KR)
(74) Representative: Viering, Jentschura & Partner mbB Patent- und Rechtsanwälte
(86) International application number: PCT/KR2019/005279
(87) International publication number: WO 2019/212262

(57) **Abstract**

A disease network construction method according to the present invention, comprises the steps of: (1) organizing cohort data in time series; (2) stratifying or grouping the data organized in step (1) by confounding variables; (3) deriving a correlation of diseases within the stratification in step (2); and (4) constructing a disease network on the basis of the correlation derived in step (3). According to the present invention, a disease may be influenced by a variety of clinical and medical confounding variables such as age, gender, race, socioeconomic variables, and regional and national health care systems, and thus a method for more reliably deriving a correlation between diseases may be provided.

## Description

### [Technical Field]

The present invention relates to a method of constructing a disease network in which hierarchization based on confounders of cohort data and an outbreak time between diseases have been considered, a method of visualizing the same, and a computer-readable recording medium in which the method has been written, and more particularly, to a method of hierarchizing cohort clinical data of patients, arranged in a time-series manner and including the data obtained from the Health Insurance Review & Assessment Service or National Health Insurance Service in Korea or the Medicare in the U.S., for each confounder, such as age, sex, race, socioeconomic variables, a medicine taken, and corresponding area of a patient, extracting, from the hierarchized data, an outbreak risk between diseases based on a relative risk and a correlation coefficient based on correlation analysis, and reconfiguring the network for each confounder by merging all cohorts, a method of visualizing hierarchization variables extracted from the link of the constructed network and a difference between outbreak times of diseases, a method of constructing a disease network in which hierarchization based on confounders of cohort data capable of visualizing various clinical variables according to the selection of each node and link in the constructed disease network and an outbreak time between diseases are considered, a method of visualizing the same, and a computer-readable recording medium in which the method has been written.

### [Background Art]

For network analysis, it is essential to secure high-quality network data. A research method related to the existing traditional network construction includes questionnaires, literature study, data mining, etc.

In particular, even in the medical field, research related to a network is actively carried out. The network includes a gene network, a protein interaction network, a medicines interaction network, a medicine-gene network, a disease interaction network, etc.

A recent issue in the search of the disease network is to visualize and analyze a sequential relationship between the outbreaks of diseases, a relative risk, directivity, a prevalence rate, an incidence, and a network by sex and for each age using clinical data. A disease may have a different aspect due to various causes depending on age, race, sex, socioeconomic variables, a medicine being taken, a geographical difference, etc. Accordingly, the question of effectiveness of a network continues to be raised even after the network is constructed because the network is configured and analyzed from a holistic viewpoint.

Furthermore, only a relative risk is monotonously used for measured values used to construct the existing clinical data-based disease network. This is a useful method in checking a disease correlation within short time monitoring, but it is preferred to construct a network by introducing the concept of time and measuring a correlation coefficient through correlation analysis, that is, more sensitive measured values, if time-series data is used.

Meanwhile, the existing disease network has considered a disease sequential relationship (directivity) and a relative risk, but does not consider a temporal difference between the outbreaks of diseases and has a problem in that a disease network is not visualized by considering several hierarchization variables.

In this aspect, it is preferred to more specifically check a disease prediction technology, a method of visualizing the same, etc. Through confounders, a temporal difference between the outbreaks of diseases, and a disease network modeling method based on correlation analysis by using a clinical disease database for patients who visit a hospital in a time-series manner for a long period.

### [Reference document]

Korean Patent Application Publication No. 10-2016-0043777 (April 22, 2016)

### [Disclosure]

### [Technical Problem]

An object of the present invention is to provide a method of constructing a disease network in which hierarchization based on confounders of cohort data and an outbreak time between diseases have been considered, wherein cohort is hierarchized using confounders, such as age, sex and age of a patient, a medication taken by a patient, corresponding area the patient resides, and sanatorium, based on the cohort big data of the patient accumulated for a long period (e.g., 10 years or more), a relative risk between diseases within the hierarchized data is calculated, a hierarchization network is preferentially constructed, and the hierarchization networks are integrated into the entire single disease network, a method of visualizing the same, and a computer-readable recording medium in which the method has been written.

Furthermore, another object of the present invention is to provide a method of constructing a disease network in which hierarchization based on confounders of cohort data and an outbreak time between diseases have been considered, wherein data is hierarchized using confounders, such as age, sex and age of a patient, a medication taken by a patient, corresponding area the patient resides, and sanatorium, based on cohort big data of the patient accumulated for a long period (e.g., 10 years or more) in a similar method, persons who have suffered from a specific disease and who have not suffered from a specific disease are classified based on data in the first year within the hierarchized data, a prevalence of a disease to be newly monitored in each group is calculated based on time series (year), a correlation between the groups is analyzed, a correlation between the specific disease and a monitored disease is checked based on a difference between two correlation coefficients, and the correlation is visualized by incorporated it into the entire single disease network, a method of visualizing the same, and a computer-readable recording medium in which the method has been written.

Furthermore, still another object of the present invention is to provide a method of constructing a disease network in which hierarchization based on confounders of cohort data and an outbreak time between diseases have been considered, wherein an average period in which one disease shifts to another disease within a population group is calculated and visualized using link information of the network, a method of visualizing the same, and a computer-readable recording medium in which the method has been written.

Furthermore, still another object of the present invention is to provide a method of constructing a disease network in which hierarchization based on confounders of cohort data and an outbreak time between diseases have been considered, wherein a disease that may newly occur in the future is predicted using time-series disease data of a patient who newly enters a cohort based on data analyzed for a constructed disease network, a visualization method, and a disease network, a method of visualizing the same, and a computer-readable recording medium in which the method has been written.

Furthermore, still another object of the present invention is to provide a method of constructing a disease network in which hierarchization based on confounders of cohort data and an outbreak time between diseases have been considered, wherein various clinical attributes of a constructed disease network are visualized, a method of visualizing the same, and a computer-readable recording medium in which the method has been written.

### [Technical Solution]

The object of the present invention is achieved by a method of constructing a disease network, including a process (1) of arranging cohort data in a time-series manner; a process (2) of hierarchizing to grouping the data arranged in the process (1) for each confounder; a process (3) of deriving a correlation between diseases within the hierarchization of in the process (2); and a process (4) of constructing a disease network based on the correlation derived in the process (3).

Furthermore, it is preferred that in the process (2), the confounder includes age, sex and age of a patient, a medication taken by a patient, corresponding area the patient resides, and sanatorium.

Furthermore, it is preferred that the process (3) includes a process of calculating a relative risk between a pre-disease and a post-disease or a process of analyzing the correlation between the diseases.

Furthermore, it is preferred that in the process (3) further includes a process of considering an outbreak period of the disease.

Furthermore, it is preferred that the cohort data includes data obtained from the Health Insurance Review & Assessment Service or National Health Insurance Service in Korea or the Medicare in the U.S.

Furthermore, it is preferred that the process (4) includes a process of constructing a subnetwork hierarchized for each confounder and a process of integrating the constructed subnetworks.

Furthermore, it is preferred that the process of integrating the subnetworks includes selecting and integrating an average value or maximum value between hierarchized groups of each subnetwork.

Meanwhile, the object of the present invention is also achieved by a method of visualizing a disease network including image visualization for visually displaying whether diseases are associated using the method of constructing a disease network.

Furthermore, it is preferred that when a user selects one or a plurality of confounders, a disease network is reconstructed in response to the user's selection, and the method includes image visualization for visually displaying whether diseases are associated based on results of the reconstruction.

On the other hand, the object of the present invention is also achieved by a computer-readable recording medium including the method of constructing a disease network or the method of visualizing a disease network.

### [Advantageous Effects]

According to the present invention, there can be provided a method of constructing a disease network in which hierarchization based on confounders of cohort data and an outbreak time between diseases have been considered, which can more reliably derive an association between diseases because a disease may be influenced by various clinical and medical confounders, such as age, sex, race, socioeconomic variables, and regional and national health care systems, a method of visualizing the same, and a computer-readable recording medium in which the method has been written.

Furthermore, there can be provided a method of deriving a single disease network by integrating hierarchized disease networks because each of the disease networks constructed as described above is results derived within each hierarchization and constructing a disease network in which hierarchization based on confounders of cohort data and an outbreak time between diseases have been considered, a method of visualizing the same, and a computer-readable recording medium in which the method has been written.

Furthermore, there can be provided a method of constructing a disease network in which hierarchization based on confounders of cohort data and an outbreak time between diseases have been considered, wherein in most of statistics methods used to construct a clinical data-based disease network, a risk of a disease is simply calculated based on a bivariate categorical form contingency table, but if time-series data for a long period can be used, an association between diseases can be checked by setting an independent variable as a year, setting a dependent variable as the number of diseases occurred, an incident rate or a prevalence, and calculating a regression coefficient based on regression analysis, a method of visualizing the same, and a computer-readable recording medium in which the method has been written.

Furthermore, there can be provided a method of constructing a disease network in which hierarchization based on confounders of cohort data and an outbreak time between diseases have been considered, wherein an average time taken from one disease to shift to another disease can be calculated, assuming that cohort data monitored for a long period is time-series data of all patients, because the time taken for one disease to shift to another disease is never considered although directivity, association strength, etc. between diseases occurred in a disease network in the past have been considered, a method of visualizing the same, and a computer-readable recording medium in which the method has been written.

Furthermore, there can be provided a method of constructing a disease network in which hierarchization based on confounders of cohort data and an outbreak time between diseases have been considered, wherein in order to improve efficient utilization to usability of a constructed disease network, more detailed information (e.g., a total number of patients, the number of patients in a disease node and link, the number of patients for each sex in the disease node and link, a ratio for each sex in the disease node and link, the number of patients for each year in the disease node and link, the number of patients for each year/sex in the disease node and link, a ratio for each year/sex in the disease node and link, the number of patients for each age in the disease node and link, the ratio of each age in the disease node and link, the number of patients for each year/age in the disease node and link, the ratio of ages for each year in the disease node and link, the number of patients for each year/sex/age in the disease node and link, the ratio of patients for each year/sex/age in the disease node and link, a prevalence for each year in the disease node and link, an incident rate for each year in the disease node and link, a distribution of medicines orally taken due to a corresponding disease in the disease node and link, a distribution of socioeconomic variables in the disease node and link, a distribution of races in the disease node and link, a distribution of regions in the disease node and link, and a distribution of sanatoriums in the disease node and link) on the corresponding node and link can be visualized and provided, a method of visualizing the same, and a computer-readable recording medium in which the method has been written.

### [Description of Drawings]

FIG. 1 is a schematic flowchart for describing a method of constructing a disease network according to an embodiment of the present invention.
FIG. 2a is an example of clinical cohort data to which an embodiment of the present invention is applied.
FIG. 2b is an example in which the data in FIG. 2a is arranged in a time-series manner.
FIG. 2c is an example in which the data in FIG. 2b is grouped for each age, that is, one of confounders.
FIG. 2d is a diagram for describing a process of calculating a relative risk in which a pre-disease affects a post-disease.
FIG. 2e is a schematic diagram for describing a relative risk calculation formula, an average value, a middle value, etc. of calculated data in FIG. 2d through the process of FIG. 2d in grouped data in FIG. 2c.
FIG. 2f is a schematic diagram for describing a process of deriving a disease correlation and period based on regression analysis of grouped data.
FIG. 3 is a table describing an example in which a disease subnetwork is constructed and a total disease network is reconstructed using a relative risk according to another embodiment of the present invention.
FIG. 4 is a graph for describing a disease subnetwork construction method using a difference between correlation coefficients.
FIG. 5 is a table and graph for describing an example in which a difference between average disease outbreak times is calculated in a disease subnetwork.
FIG. 6 is a schematic diagram for describing that various clinical variables are visualized in all disease networks.

### [Mode for Invention]

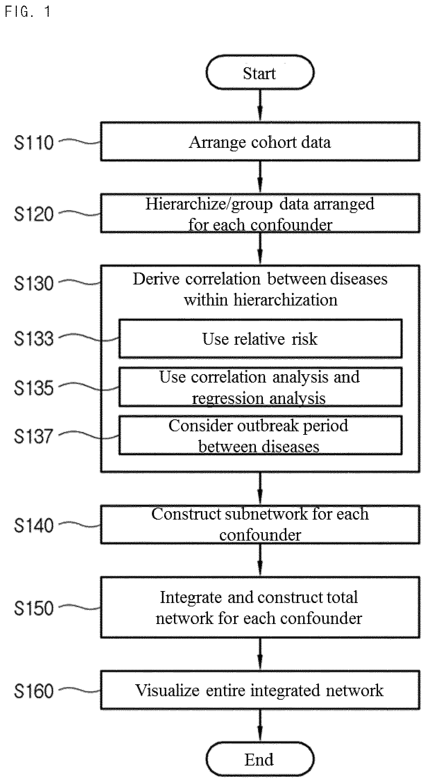

A method of constructing a disease network in which hierarchization based on confounders of cohort data and an outbreak time between diseases have been considered (hereinafter referred to as a "disease network construction method") according to the present invention, a method of visualizing the same, and a computer-readable recording medium in which the method has been written are described in detail with reference to FIGS. 1 to 6.

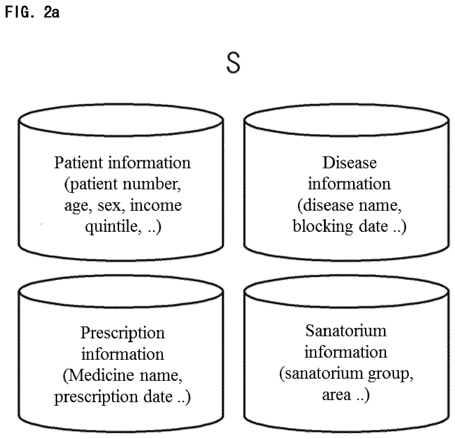

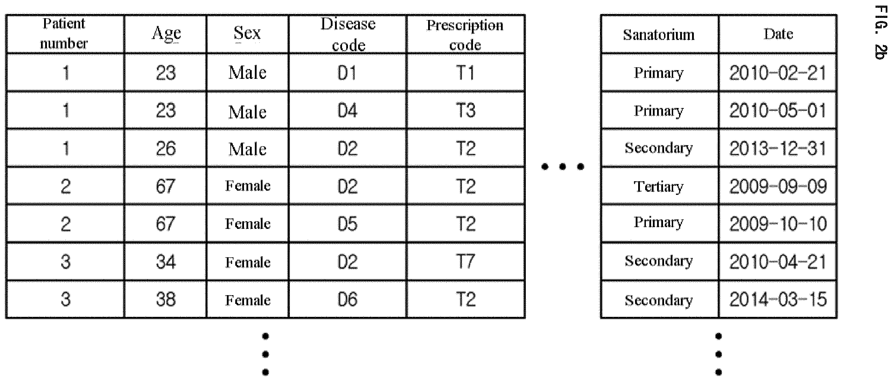

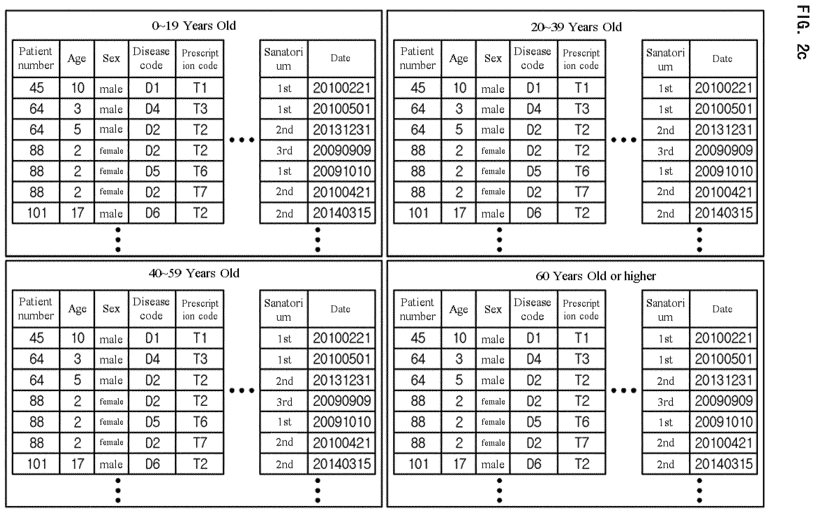

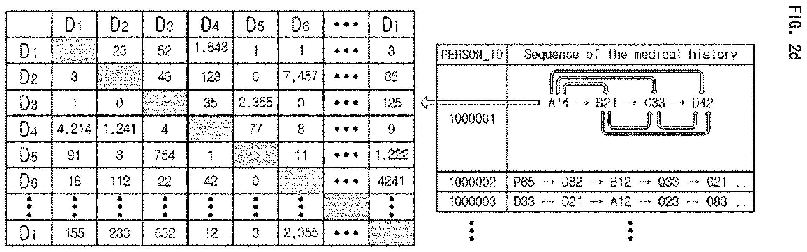

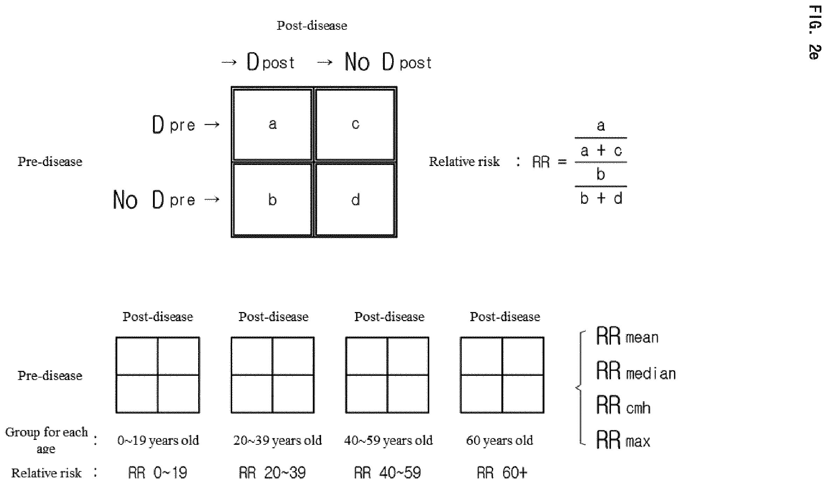

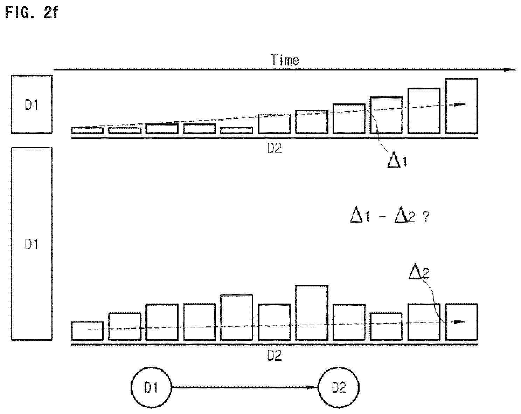

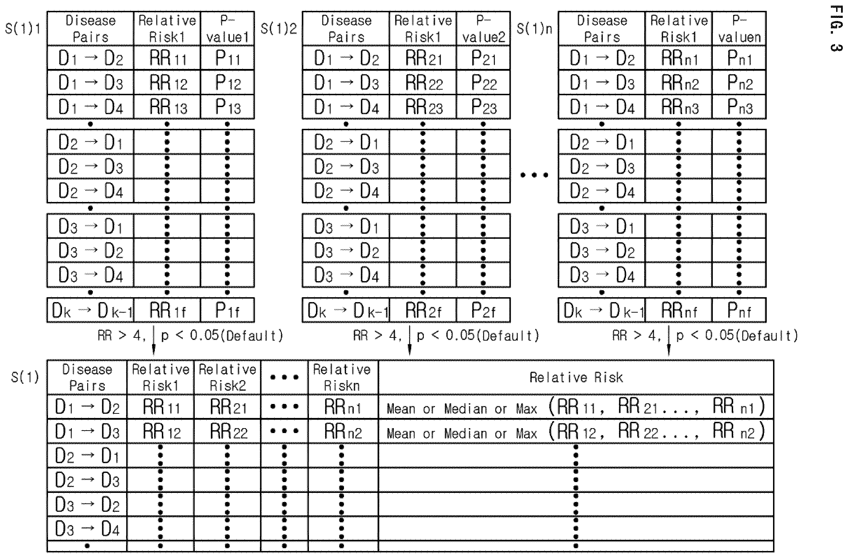

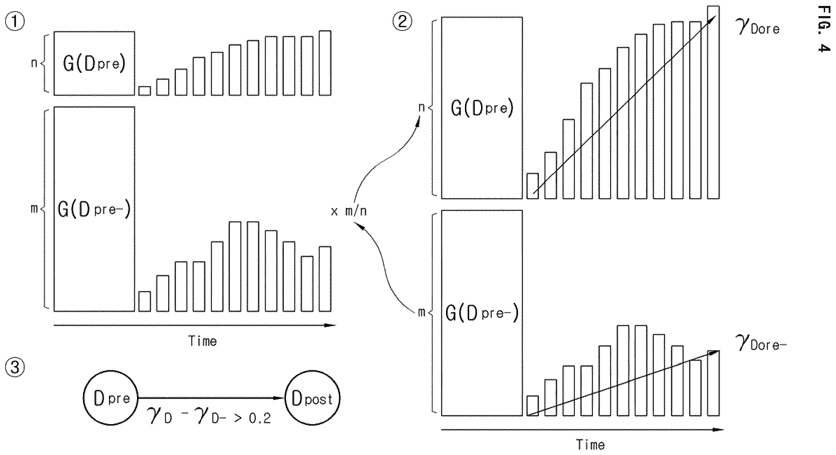

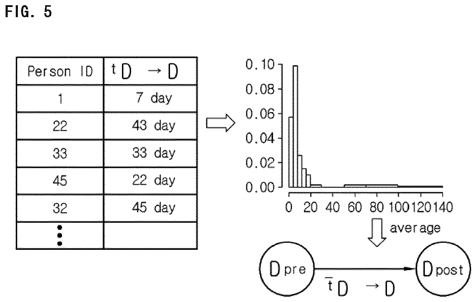

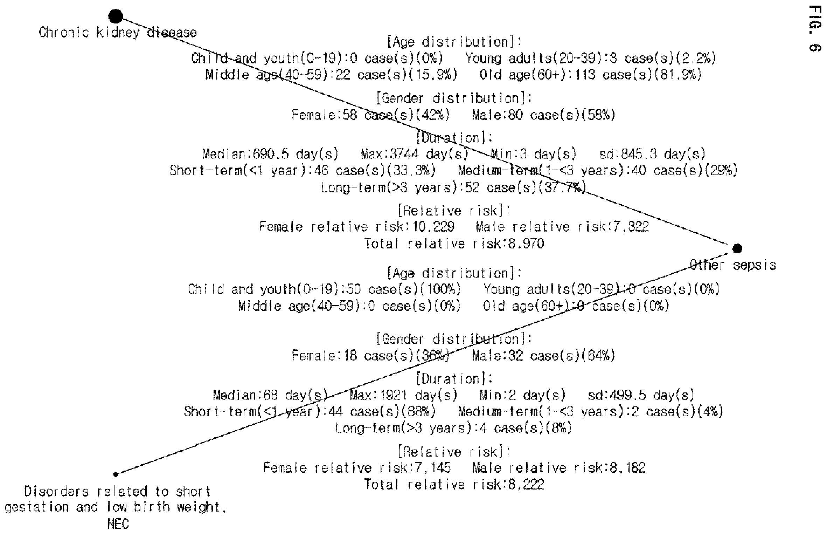

FIG. 1 is a schematic flowchart for describing a method of constructing a disease network according to an embodiment of the present invention. FIG. 2a is an example of clinical cohort data to which an embodiment of the present invention is applied. FIG. 2b is an example in which the data in FIG. 2 is arranged in a time-series manner. FIG. 2c is an example in which the data in FIG. 2b is grouped for each age, that is, one of confounders. FIG. 2d is a diagram for describing a process of calculating a relative risk in which a pre-disease affects a post-disease. FIG. 2e is a schematic diagram for describing a relative risk calculation formula, an average value, a middle value, etc. of calculated data in FIG. 2d through the process of FIG. 2d in grouped data in FIG. 2c. FIG. 2f is a schematic diagram for describing a process of deriving a disease correlation and period based on regression analysis of grouped data. FIG. 3 is a table describing an example in which a disease subnetwork is constructed and a total disease network is reconstructed using a relative risk according to another embodiment of the present invention. FIG. 4 is a graph for describing a disease subnetwork construction method using a difference between correlation coefficients. FIG. 5 is a table and graph for describing an example in which a difference between average disease outbreak times is calculated in a disease subnetwork. FIG. 6 is a schematic diagram for describing that various clinical variables are visualized in all disease networks.

The disease network construction method according to the present invention, as shown in FIGS. 1 to 6, preferably includes a process (1) S110 of arranging cohort data in a time-series manner; a process (2) S120 of hierarchizing to grouping the data arranged in the process (1) for each confounder; a process (3) S130 of deriving a correlation between diseases within the hierarchization of the process (2); a process (4) S140 and S150 of constructing a disease network based on the correlation derived in the process (3); and a process S160 of visualizing a disease network method.

First, all cohort data S, such as FIG. 2a, are arranged in a time-series manner as in FIG. 2b (S110).

Next, the data arranged in a time-series manner is hierarchized to grouped for each confounder (e.g., S(l), S(2), S(3) ..., S(n)) or for each combination of every two or more confounders (e.g., S(1,2), S(1,3), ..., S(1,2,3) ..., S(1,2,..n)) (S120).

For another example, if all the cohort data S is hierarchized based on an age, that is, a confounder, as shown in FIG. 2c, S (age) 1 may be 19 years old or less, S (age) 2 may be 20-39 years old, S (age) 3 may be 40-59 years old, and S (age) 4 may be the 60s or more. Furthermore, if all the cohort data S is hierarchized into subgroups by sex, that is, another confounder, all the cohort data S becomes two subgroups, that is, S (sex) 1 of a male and S (sex) 2 of a female.

Accordingly, if all the cohort data S is hierarchized and classified into subgroups for each age and sex, that is, all the cohort data S may be theoretically classified into eight subgroups, that is, two confounders, S (age, sex) 1 is 19 years old or less and a male, S (age, sex) 2 is 20∼39 years old and a male, S (age, sex) 3 is 40∼59 years old and a male, S (age, sex) 4 is 60 years old or higher, S (age, sex) 5 is 19 years old or less and a female, S (age, sex) 6 is 20∼39 years old and a female, S (age, sex) 7 is 40∼59 years old and a female, and S (age, sex) 8 is 60 years old or higher and a female. Thereafter, steps may be divided into the step of extracting a disease subnetwork from each subgroup using a statistical method and the step of reconstructing all disease networks based on hierarchization variables from each extracted disease subnetwork.

In this case, the confounder may include an age, sex, socioeconomic variables (to position), a corresponding (residence) area, a race, a sanatorium, etc. of a patient. In an example in which the confounders are hierarchized and grouped for each age, as shown in FIG. 2c, the confounders may be divided into four groups of 0-19 years old, 20-39 years old, 40-59 years old, and 60 years old or higher. Hereinafter, for convenience of description, an example in which ages are hierarchized is described. One or a plurality of such confounders may be selected as described above.

The process S130 of constructing a disease subnetwork within a subgroup configured for each confounder (or a combination of confounders) may include a process S133 of deriving a disease correlation based on a relative risk between a pre-disease and a post-disease and a process S135 of deriving a disease correlation based on correlation analysis between diseases.

First, in the process of deriving a disease correlation based on a relative risk, as shown in FIGS. 2d and 3, a disease network may be constructed based on clinical data of a cohort. A description of such an example is hereinafter omitted because the example was applied as "Disease Network Construction Method considering Stratification according to confounding factors of Cohort data and Disease occurring time between the diseases, Its Visualization Method and Computer Readable Record Medium thereof (Korean Patent Application No. 10-016-0163260)" including the present inventor. One disease subnetwork for a specific group of a confounder is produced because such a disease network is constructed only within a specific subgroup based on a confounder or a combination of confounders.

As shown in FIGS. 2d, 2e and 3, all the cohort data S may be divided into subgroups for each specific confounder (or a combination of confounders), and a disease subnetwork may be extracted based on a relative risk in each subgroup. For example, a case where the relative risk is more than 4 and significance (P-value) is less than 0.05 may be extracted by default. In this case, if a user selects and sets the relative risk and the P-value, a disease subnetwork in which directivity is considered may be extracted based on the set values. That is, if the number of subgroups of S(l) is n, n disease subnetworks may be extracted (refer to FIG. 3).

Furthermore, all the cohort data S is divided into subgroups for each specific confounder. In order to extract a disease subnetwork from each subgroup based on correlation analysis, a group that has suffered from a specific disease D pre if once during a monitoring period of an initial 1 year in a pre-disease D pre and a post-disease D post is set as G(D pre), and a group that has never suffered from the specific disease D pre is set as G(D pre-) (refer to FIG. 4). Next, the number of persons accumulated by year (or a prevalence rate or cumulative incidence) in which the post-disease D post has occurred in each group of G(D pre) and G(D pre-) from the second year to the last year including data is calculated. Assuming that G(D pre) = n for the initial 1 year and G(D pre-) = m, in order to correct an error occurring due to a scaling difference, the number of accumulated persons by each year that belong to the G(D pre) group is multiplied by m/n, an x axis is set as the year, a y axis is set as the number of accumulated persons of G(D pre) and G(D pre-), and correlation analysis is then performed (refer to FIG. 4). As the results of the correlation analysis, two correlation coefficient rD pre and rD pre- and p-values (P Dpre, P Dpre-), that is, two significance level values, are calculated. Only a case of r Dpre-> 0 is taken regardless of values of r Dpre > 0 and P Dpre-, wherein P Dpre < 0.05. Whether the disease D pre affects the disease D post is evaluated by calculating a difference (r Dpre - r Dpre-). Theoretically, the range of rD pre - r Dpre- is -1 ∼ 1. Assuming that only a case where r Dpre - r Dpre- > 0.2 is a "positive correlation" designated by default, a connection "D pre → D post" is performed. Through such a method, a subnetwork may be constructed for each confounder by connecting a disease pair having a meaningful positive correlation to all disease pairs of all subgroups by sequentially applying {D 1, D 2, D 3,... D k} in each subgroup (S140) . If a user randomly designates a value of r Dpre - r Dpre-, a value of more than the designated value may be extracted as a positive correlation.

Furthermore, alternatively, a figure schematically showing a process of deriving a linear change through regression analysis between diseases is FIG. 2f.

On the other hand, another embodiment of the present invention includes a process S137 of deriving and databasing an average outbreak period from a pre-disease to a post-disease, and can effectively visualize a disease network. To this end, as shown in FIG. 5, first, after an outbreak time t Dpre → Dpost of each person, that is, D pre → D post, for each of subgroups having a specific hierarchization variable is calculated, an average outbreak time t' Dpre → Dpost of the persons is calculated. Through such a method, information related to a period in a subnetwork hierarchized for each confounder may be constructed by sequentially applying {D 1, D 2, D 3,... D k} to each group and calculating an average outbreak time of disease pairs with respect to all disease pairs.

The relative risk of a disease pair, the difference between correlation coefficients, and the average outbreak time are values calculated within a specific group for each hierarchization variable. A method S150 of integrating the relative risk, the difference and the average outbreak time with respect to all groups not a specific group preferably includes a method of calculating and determining the mean between the groups and a method of selecting a maximum value in each group.

A method of integrating disease networks for a relative risk may be provided to the two methods (the methods of selecting an average value and a maximum value). The mean between groups for a relative risk may include a method using the arithmetic mean of each group for a relative risk and a method using a middle value. Furthermore, a maximum value among groups is selected in each disease and provided as the relative risk. This fully depends on a user's selection, and the mean between groups is provided by default.

Furthermore, the method of integrating disease networks based on a difference between correlation coefficients may also be provided to the two methods (the methods of selecting an average value and a maximum value). The mean between groups based on a difference between correlation coefficients provides a method using the arithmetic mean of differences between correlation coefficients of groups and a method using a middle value. Furthermore, a maximum value among groups is selected in each disease and provided as a difference between correlation coefficients. This fully depends on a user's selection, and the mean between groups is provided by default.

Furthermore, an average outbreak period between disease pairs may also be provided to the two methods (the methods of selecting an average value and a maximum value). The mean between groups based on an average outbreak period between disease pairs provides a method using the arithmetic mean of average outbreak periods between disease pairs of each group and a method using a middle value. Furthermore, a maximum value among groups is selected and provided as a difference between average outbreak periods of disease pairs. This fully depends on a user's selection, and the mean between groups is provided by default.

A figure showing an example of the disease network integrated and constructed as described above is FIG. 2h.

The disease network constructed as described above may be visualized (S160) so that a user can easily check and view the disease network at a look. According to the present invention, such a disease network may be reconstructed and displayed based on a confounder randomly set by a user. The confounder includes sex, an age, a social layer factor (e.g., income), a race, a corresponding area, a sanatorium, etc. of a patient. For example, if a user selects an age and sets three subgroups (29 years old or less, 30∼59 years old, and 59 years old or higher) depending on the age, all disease networks determined by the three subgroup are reconstructed based on the age. For another example, if a user selects an age and sex at the same time and sets six subgroups (a male of 29 years old or less, a female of 29 years old or less, a male of 30-59 years old, a female of 30-59 years old, a male of 60 years old or higher, and a female of 60 years old or higher), all disease networks determined by the six subgroups are preferably reconstructed and visualized.

In all the disease networks, a node may be represented as a circle and a square. The size of the node is determined by a relative prevalence rate or incidence of diseases. It is preferred that in the visualization mode of a link in a disease network, visualization is possible based on a mode selected by a user, among a link mode based on a relative risk and a link mode based on a correlation coefficient. Furthermore, when selecting the mode of a link, a user may select whether visualization is visualized based on the arithmetic mean or visualization based on a middle value. A link may be visualized based on a maximum value. For example, if a user wants to see a relative risk for each age in a disease pair, all the relative risks of disease pairs in each subgroup are visualized and displayed. If the user selects a relative risk for each age and sex in the disease pair, all the relative risks of the disease pairs in each subgroup may be visualized and displayed. Likewise, a relative risk may be displayed with respect to a combination of all hierarchization variables set by a user.

Likewise, if a user wants to view a difference between correlation coefficients for each age in a disease pair, a difference between all the correlation coefficients of disease pairs in each subgroup is visualized and displayed. If the user wants to view a difference between the correlation coefficients for each age and sex in the disease pair, a difference between all the correlation coefficients of the disease pairs in each subgroup is visualized and displayed. Likewise, a difference between the correlation coefficients may be calculated and displayed with respect to a combination of all confounders set by a user.

Furthermore, in order to visualize a difference between outbreak times of disease pairs in a disease network, in a single disease network, it is preferred that a link is classified into blue to red and visualized from a short term to a long term by representing a link having a short term (short-Term) as blue, a link having a middle term (Middle-Term) as green, and a link having a long term (Long-Term) as red. A criterion for dividing a difference between outbreak times of disease pairs may be selected by a user.

Furthermore, if a specific node or link is selected in the constructed disease network, more detailed information on corresponding disease information may be visualized in a dashboard form. In detailed information on a disease node, it is preferred that first, a total number of patients included in data is visualized, second, the number of patients included in the data is visualized by year in a bar graph form, third, a total number of patients corresponding to a disease node are visualized, fourth, the number and ratio of patients for each sex, corresponding to a disease node, is visualized, fifth, the number of patients for each year in a disease node is visualized in a bar graph form, sixth, the number and ratio of patients for each year and sex in a disease node is visualized in a bar graph form, seventh, the number of patients for each age in a disease node is visualized in a bar graph form, eighth, a ratio for each age in a disease node is visualized in a pie chart form, ninth, the number of patients for each year-age in a disease node is visualized in a bar graph form, tenth, the ratio of ages for each year in a disease node is visualized in a bar graph form, eleventh, the number of patients and ratio for each year-sex-age in a disease node is visualized in a bar graph form, twelfth, a prevalence for each year in a disease node is visualized in a bar graph form, thirteenth, an incident rate and accumulated incident rate for each year in a disease node is visualized in a bar graph form, fourteenth, a distribution of medicines taken orally due to a corresponding disease in a disease node is visualized in a pie chart form, fifteenth, a distribution according to socioeconomic variables in a disease node is visualized in a pie chart form, sixteenth, a distribution of races in a disease node is visualized in a pie chart form, seventeenth, a distribution of regions in a disease node is visualized in a map, eighteenth, and a distribution for each sanatorium (primary, secondary, and tertiary hospitals) in a disease node is visualized in a pie chart form.

In the detailed information on a link between disease pairs, it is preferred that first, a relative risk, a difference between regression coefficients, and an average outbreak period between disease pairs are visualized, second, a total number of patients corresponding to a link between diseases is visualized, third, the number and ratio of patients for each sex corresponding to a link between diseases is visualized, fourth, the number of patients for each year in a link between diseases is visualized in a bar graph form, fifth, the number and ratio of patients for each year and sex in a link between diseases is visualized in a bar graph form, sixth, the number of patients for each age in a link between diseases is visualized in a bar graph form, seventh, a ratio for each age in a link between diseases is visualized in a pie chart form, eighth, the number of patients for each year-age in a link between diseases is visualized in a bar graph form, ninth, the ratio of ages for each year in a link between diseases is visualized in a bar graph form, tenth, the number of patients and ratio for each year-sex-age in a link between diseases is visualized in a bar graph form, eleventh, a prevalence for each year in a link between diseases is visualized in a bar graph form, twelfth, an incident rate for each year and an accumulated incident rate in a link between diseases is visualized in a bar graph form, thirteenth, a distribution of medicines orally taken due to a corresponding disease in a link between diseases is visualized in a pie chart form, fourteenth, a distribution according to socioeconomic variables in a link between diseases is visualized in a pie chart form, fifteenth, a distribution of races in a link between diseases is visualized in a pie chart form, sixteenth, a distribution of regions in a link between diseases is visualized in a map, seventeenth, a distribution of sanatoriums (primary, secondary, and tertiary hospitals) in a link between diseases is visualized in a pie chart form. An example similar to the detailed information is shown in FIG. 6.

It is preferred that an example of a list for visualizing the disease network includes a total number of patients, the number of patients in a disease node and link, the number of patients for each sex in a disease node and link, a ratio for each sex in a disease node and link, the number of patients for each year in a disease node and link, the number of patients for each year and sex in a disease node and link , a ratio for each year and sex in a disease node and link, the number of patients for each age in a disease node and link, a ratio for each age in a disease node and link, the number of patients for each year and age in a disease node and link, the ratio of ages for each year in a disease node and link, the number of patients for each year-sex-age in a disease node and link, the ratio of patients for each year-sex-age in a disease node and link, a prevalence for each year in a disease node and link, an incident rate for each year in a disease node and link, a distribution of medicines orally taken due to a corresponding disease in a disease node and link, a distribution of socioeconomic variables in a disease node and link, a distribution of races in a disease node and link, a distribution of regions in a disease node and link, and a distribution of sanatoriums in a disease node and link, an average outbreak period in a disease node link, etc.

On the other hand, the object of the present invention is also achieved by a computer-readable recording medium in which the method of constructing a disease outbreak network and the method of visualizing the same has been written.

Furthermore, it is preferred that the cohort data includes data obtained from the Health Insurance Review & Assessment Service or National Health Insurance Service in Korea or the Medicare in the U.S.

As described above, data is hierarchized using several confounders (e.g., for each age, sex, race, socioeconomic variable, medicine being taken, corresponding (residence) area, and sanatorium of a patient) from cohort data of a patient arranged in time series. A disease subnetwork in which an average outbreak period between diseases is considered is constructed based on the hierarchized data using the method including a relative risk and correlation analysis. A total network is produced by reconstructing the subnetworks for each hierarchization variable. When a user finally selects a disease node or link by considering convenience, detailed additional information on the node and link may be extracted and visualized.

According to the present invention, there can be provided a method of constructing a disease network in which hierarchization based on confounders of cohort data and an outbreak time between diseases have been considered, which can more reliably derive an association between diseases because a disease may be influenced by various clinical and medical confounders, such as age, sex, race, socioeconomic variables, and regional and national health care systems, a method of visualizing the same, and a computer-readable recording medium in which the method has been written.

Furthermore, there can be provided a method of deriving a single disease network by integrating hierarchized disease networks because each of the disease networks constructed as described above is results derived within each hierarchization and constructing a disease network in which hierarchization based on confounders of cohort data and an outbreak time between diseases have been considered, a method of visualizing the same, and a computer-readable recording medium in which the method has been written.

Furthermore, there can be provided a method of constructing a disease network in which hierarchization based on confounders of cohort data and an outbreak time between diseases have been considered, wherein in most of statistics methods used to construct a clinical data-based disease network, a risk of a disease is simply calculated based on a bivariate categorical form contingency table, but if time-series data for a long period can be used, an association between diseases can be checked by setting an independent variable as a year, setting a dependent variable as the number of diseases occurred, an incident rate or a prevalence, and calculating a regression coefficient based on regression analysis, a method of visualizing the same, and a computer-readable recording medium in which the method has been written.

Furthermore, there can be provided a method of constructing a disease network in which hierarchization based on confounders of cohort data and an outbreak time between diseases have been considered, wherein an average time taken from one disease to shift to another disease can be calculated, assuming that cohort data monitored for a long period is time-series data of all patients, because the time taken for one disease to shift to another disease is never considered although directivity, association strength, etc. between diseases occurred in a disease network in the past have been considered, a method of visualizing the same, and a computer-readable recording medium in which the method has been written.

Furthermore, there can be provided a method of constructing a disease network in which hierarchization based on confounders of cohort data and an outbreak time between diseases have been considered, wherein in order to improve efficient utilization to usability of a constructed disease network, more detailed information (e.g., a total number of patients, the number of patients in a disease node and link, the number of patients for each sex in the disease node and link, a ratio for each sex in the disease node and link, the number of patients for each year in the disease node and link, the number of patients for each year/sex in the disease node and link, a ratio for each year/sex in the disease node and link, the number of patients for each age in the disease node and link, the ratio of each age in the disease node and link, the number of patients for each year/age in the disease node and link, the ratio of ages for each year in the disease node and link, the number of patients for each year/sex/age in the disease node and link, the ratio of patients for each year/sex/age in the disease node and link, a prevalence for each year in the disease node and link, an incident rate for each year in the disease node and link, a distribution of medicines orally taken due to a corresponding disease in the disease node and link, a distribution of socioeconomic variables in the disease node and link, a distribution of races in the disease node and link, a distribution of regions in the disease node and link, and a distribution of sanatoriums in the disease node and link) on the corresponding node and link can be visualized and provided, a method of visualizing the same, and a computer-readable recording medium in which the method has been written.

In this case, although an embodiment of the present invention has been shown and described, those skilled in the art to which the present invention pertains may undestand that the present embodiment may be modified withouth departing from the principle or spirit of the present invention. Accordingly, the scope of rights of the present invention may be determined by the attached claims and equivalents thereof.

### [industrial Applicability]

According to the present invention, there can be provided a method of constructing a disease network in which hierarchization based on confounders of cohort data and an outbreak time between diseases have been considered, which can more reliably derive an association between diseases because a disease may be influenced by various clinical and medical confounders, such as age, sex, race, socioeconomic variables, and regional and national health care systems, a method of visualizing the same, and a computer-readable recording medium in which the method has been written.

Furthermore, there can be provided a method of deriving a single disease network by integrating hierarchized disease networks because each of the disease networks constructed as described above is results derived within each hierarchization and constructing a disease network in which hierarchization based on confounders of cohort data and an outbreak time between diseases have been considered, a method of visualizing the same, and a computer-readable recording medium in which the method has been written.

Furthermore, there can be provided a method of constructing a disease network in which hierarchization based on confounders of cohort data and an outbreak time between diseases have been considered, wherein in most of statistics methods used to construct a clinical data-based disease network, a risk of a disease is simply calculated based on a bivariate categorical form contingency table, but if time-series data for a long period can be used, an association between diseases can be checked by setting an independent variable as a year, setting a dependent variable as the number of diseases occurred, an incident rate or a prevalence, and calculating a regression coefficient based on regression analysis, a method of visualizing the same, and a computer-readable recording medium in which the method has been written.

Furthermore, there can be provided a method of constructing a disease network in which hierarchization based on confounders of cohort data and an outbreak time between diseases have been considered, wherein an average time taken from one disease to shift to another disease can be calculated, assuming that cohort data monitored for a long period is time-series data of all patients, because the time taken for one disease to shift to another disease is never considered although directivity, association strength, etc. between diseases occurred in a disease network in the past have been considered, a method of visualizing the same, and a computer-readable recording medium in which the method has been written.

Furthermore, there can be provided a method of constructing a disease network in which hierarchization based on confounders of cohort data and an outbreak time between diseases have been considered, wherein in order to improve efficient utilization to usability of a constructed disease network, more detailed information (e.g., a total number of patients, the number of patients in a disease node and link, the number of patients for each sex in the disease node and link, a ratio for each sex in the disease node and link, the number of patients for each year in the disease node and link, the number of patients for each year/sex in the disease node and link, a ratio for each year/sex in the disease node and link, the number of patients for each age in the disease node and link, the ratio of each age in the disease node and link, the number of patients for each year/age in the disease node and link, the ratio of ages for each year in the disease node and link, the number of patients for each year/sex/age in the disease node and link, the ratio of patients for each year/sex/age in the disease node and link, a prevalence for each year in the disease node and link, an incident rate for each year in the disease node and link, a distribution of medicines orally taken due to a corresponding disease in the disease node and link, a distribution of socioeconomic variables in the disease node and link, a distribution of races in the disease node and link, a distribution of regions in the disease node and link, and a distribution of sanatoriums in the disease node and link) on the corresponding node and link can be visualized and provided, a method of visualizing the same, and a computer-readable recording medium in which the method has been written.

## Claims

1. A method of constructing a disease network, comprising:
a process (1) arranging cohort data in a time-series manner;
a process (2) hierarchizing to grouping the data arranged in the process (1) for each confounder;
a process (3) deriving a correlation between diseases within the hierarchization of in the process (2); and
a process (4) constructing a disease network based on the correlation derived in the process (3).

2. The method of claim 1, wherein in the process (2), the confounder comprises an age, sex and age of a patient, a medication taken by a patient, corresponding area the patient resides, and sanatorium.

3. The method of claim 1 or 2, wherein the process (3) comprises:
a process of calculating a relative risk between a pre-disease and a post-disease or a process of analyzing the correlation between the diseases.

4. The method of claim 3, wherein in the process (3) further comprises a process of considering an outbreak period of the disease.

5. The method of claim 1, wherein the cohort data comprises data obtained from Health Insurance Corporation and Health Insurance Review Board, Medicare Data of the U.S.A or common data model (CDM)-based data of International Odyssey Consortium for medical big data sharing.

6. The method of claim 1, wherein the process (4) comprises
a process of constructing a subnetwork hierarchized for each confounder, and
a process of integrating the constructed subnetworks.

7. The method of claim 6, wherein the process of integrating the subnetworks comprises selecting and integrating an average value or a maximum value between hierarchized groups of each subnetwork.

8. A method of visualizing a disease network comprising image visualization for visually displaying whether diseases are associated using the method of constructing a disease network according to claim 1.

9. The method of claim 8, wherein:
when a user selects one or a plurality of confounders, a disease network is reconstructed in response to the user's selection, and
the method comprises image visualization for visually displaying whether diseases are associated based on results of the reconstruction.

10. A computer-readable recording medium comprising the method of constructing a disease network according to claim 1 or the method of visualizing a disease network according to claim 8.
